# EUROPEAN PATENT SPECIFICATION

(11) **EP 0 876 801 B1**
(45) Date of publication and mention of the grant of the patent: **10.09.2003**
(21) Application number: 98830054.7
(22) Date of filing: 09.02.1998
(51) Int. Cl.: A61C 7/14

(54) **Orthodontic bracket**
Orthodontischer Halter
Bracket orthodontique

(30) Priority: 06.05.1997 IT FI970066 U
(43) Date of publication of application: 11.11.1998
(73) Proprietor: Leone S.p.A., 50019 Sesto Fiorentino (Firenze) (IT)
(72) Inventor:
(74) Representative: Martini, Lazzaro

(56) References cited:
- US-A- 5 326 259
- US-A- 5 595 484
- US-A- 5 622 494

## Description

The present invention relates to an orthodontic aid or bracket.

It is known that the orthodontic aids used for correcting dental malformations basically consist of one or more bodies featuring seats for an orthodontic archwire in their inner part, which are wedded to a retention base, as to allow various operating orthodontic aids to be suitable for the same archiwire which extends along the arch subjected to therapeutical treatment.

The manufacturing of these orthodontic aids is diversified on account of the different shape of each tooth so that a single orthodontic aid corresponds to a respective tooth. For example, an orthodontic aid for the upper right eye-tooth is different from that for the upper left eye-tooth and so on.

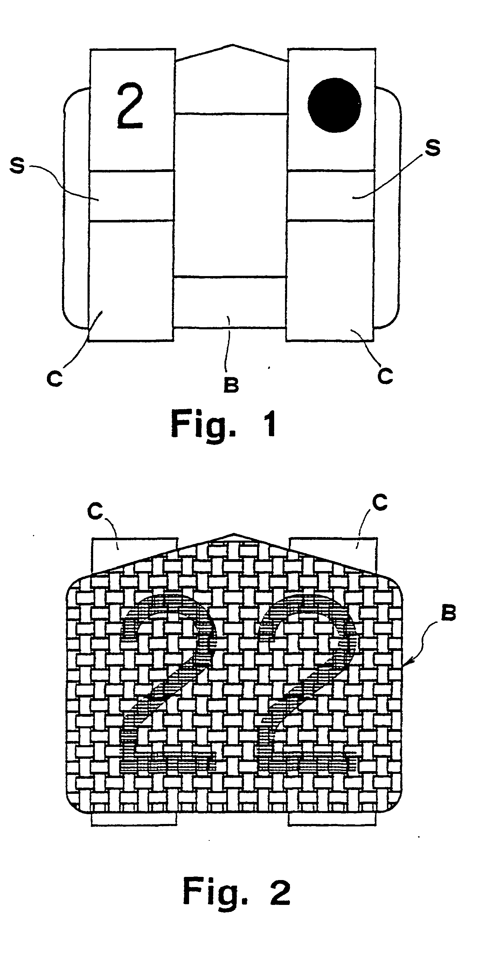

At present, in order to identify orthodontic aids in relation to the teeth for which they are suitable, each orthodontic aid is usually marked with a corresponding sign which consists of a numerical abbreviation or of a colour dot positioned on the disto-gingival edge of the orthodontic aid as is clearly shown in fig. 1 of the enclosed drawings wherein said codes consist of a "2" and of a black dot foreseen on the tie wings (A) of the bodies wedded to the retention base (B). However, an identification code positioned on this edge of the orthodontic aid consequently becomes less perceptible and recognizable, being the surface available, the area of which is generally about 1 square millimeter, quite limited, which can cause tiredness and mistakes on the part of the orthodontist.

The US patent no. 5326259 describes a method of manufacturing orthodontic aids with identifying indicia which can be positioned on the upper area of the bodies wedded to the retention base. But, despite its complexity, this common operative method presents the above mentioned disadvantages.

The US patent no. 5622494 relates to a plastic orthodontic appliance having projecting structure extending outwardly from a bonding base which is shaped to have a bonding area within the bonding base and the free edge of the projecting structure. A portion of the bonding base may be an open area without projections to allow for the molding of an identifying mark.

However, this known appliance doesn't allow for molding identifying marks easily recognizable by the orthodontist. In fact, by increasing the marking area the bonding area will be correspondingly reduced with a relevant bonding action reduction. Furthermore, the molding of identifying marks is cause for a limited possibility of choise for the appliance bonding structure, said molding technique being not utilizable, for example, in case of brackets having bonding net-like structure, which are greatly reliable and fully tested, due to the fact that the molding techinique would imply a deformation of such entity to functionally damage the bonding net-like structure.

The US patent no. 5595484 describes a plastic aesthetical orthodontic bracket. The retention base may be provided with identification marks which are molded in small recessed portions of the retention base to be not noticeable when the bracket is bonded to the relevant tooth. The molding of the identification marks in the small recesses of the lingual surface of the bracket may involve an undue visual weariness by part of the orthodontist because said marks are too small. Even in this case a bracket having a retention base with net-like structure is not utilizable because the molding techinique would imply an untollerable deformation of the net-like structure.

The object underlying the present invention is to overcome the aforesaid disadvantages.

This object has been achieved in accordance with the invention by manufacturing an orthodontic bracket having the features as described in claim 1. Further features of the invention are the subject of the dependent claims.

The present invention has the advantage of rendering the identification code more easily recognizable in every operative condition without modifying the structure of the orthodontic aid and improving its hold while it is being used, all this in an easy, reliable and cheap manner.
Furthermore, the anchorage of the retention base to the tooth is not adversely affected, contrarly to the case of brackets having molded identification marks, since the thickness of the identification marks according to the invention is substantially null.

A further advantage lies in that the marking may be carried out even separately from the manufacturing or assemblage of the bracket.

These and further advantages and characteristics of the present invention will be better understood by anyone skilled in the art from a reading of the following description in conjunction with the attached drawings, given as a practical exemplification of the invention, but not to be considered in a limitative sense, wherein:
- Fig. 1 shows a view of an orthodontic aid in its interity with traditional identification codes;
- Fig. 2 shows a back view of an orthodontic aid in accordance with the invention.

An orthodontic bracket in its essential strucuture and with reference to the figures of the enclosed drawings, in accordance with the invention, consists of a retention base (B) and of two bodies (C) wedded to the retention base and provided with seats for a corresponding section of an orthodontic archwire. The orthodontic aid is provided with indicia consisting of a numerical code ("22" in fig. 2) positioned on the back side of the retention base (B) that is to say on the side which will adhere to the tooth. In the example of fig. 2, the code of the orthodontical aid corresponds to the position of the tooth for which it is suitable, in compliance with the "FDI SISTEM" identification system (i.e. FEDERATION DENTAIRE INTERNATIONALE) wherein teeth can be identified thanks to a progressive numeration with two figures, always starting from the medium line, wherein the first ten indicates the upper right side, the second ten indicates the upper left side, the third ten indicates the lower left side and the fourth ten indicates the lower right side, as is shown in the chart below:

| | | | | | | | | | | | | | | | |
|---|---|---|---|---|---|---|---|---|---|---|---|---|---|---|---|
| 18 | 17 | 16 | 15 | 14 | 13 | 12 | 11 | 21 | 22 | 23 | 24 | 25 | 26 | 27 | 28 |
| 48 | 47 | 46 | 45 | 44 | 43 | 42 | 41 | 31 | 32 | 33 | 34 | 35 | 36 | 37 | 38 |

In this way, for example, the lower central incisors are identified by the numbers 41 and 31 and son on.
Any other identification system of the position of teeth can advantageously serve the purpose as well. To better explain this system and in accordance with a technique already known to technicians operating in this field, the aforementioned identification code may consist of a number with one figure identifying the tooth, proceded or followed by a "+" or a "-" sign, which indicates if the number belongs to the right or to the left side: the 3 + symbol identifies the upper right eye-tooth and the - 4 symbol identifies the first lower left premolar. Anyway, whatever symbols may be chosen, a much wider marking area, which can even be 9 square millimeter, is found in the tergal base section (B), so the code size will consequently correspond to that of this area, as to allow the orthodontist to better recognize it, to avoid getting tired and making mistakes and to execute the operation in a safe manner.

The marking of the retention base (B) can be produced by means of a laser apparatus which many technicians already know. The orthodontic aid illustrated in fig. 2 features a net-like retention base. The anchorage of the retention base (B) to the tooth can be effected in the usual manner, by applying adhesive or dental cement on the dental surface, which has previously been treated with acids facilitating the formation of microscopic leaks for a consequent better retention.

The marking produced in accordance with the invention, by means of a laser beam, allows the formation of a much wider area where adhesive can operate more effectively.
The marking of orthodontic aids in accordance with the invention can be produced along with the traditional marking by positioning said identification code both on the back and on the front side of these.

## Claims

1. Orthodontic bracket provided with a retention base (B) for a respective tooth and identifying indicia in relation to the tooth for which the orthodontic aid is suitable, said indicia consisting of a sign foreseen on the back side of said retention base (B), **characterized in that** said retention base involves a marking area of at least 3 square millimeters, is that said sign is produced by means of a laser apparatus and extends across the net-like structure of the retention base.

2. Orthodontic bracket according to claim 1 **characterized by** said sign consisting of a number with two figures.

3. Orthodontic bracket according to claim 1 **characterised by** said sign consisting of a number with one figure preceded or followed either by the "+" or by the"-" sign.

## Patentansprüche

1. Zahnregulierungsspange, die mit einer Haltebasis (B) für einen entsprechenden Zahn und Identifizierungsmarkierungen bezüglich des Zahns versehen ist, für den die Zahnregulierungshilfe geeignet ist, wobei die Kennzeichnung aus einem auf der Rückseite der Haltebasis (B) vorgesehene Zeichen besteht, **dadurch gekennzeichnet, dass** die Haltebasis eine Markierungsfläche von wenigstens drei mm² umfasst, dass das Zeichen mit Hilfe einer Lasereinrichtung erzeugt ist und sich über die netzartige Struktur der Haltebasis erstreckt.

2. Zahnregulierungsspange nach Anspruch 1, **dadurch gekennzeichnet, dass** das Zeichen aus einer Zahl mit zwei Ziffern besteht.

3. Zahnregulierungsspange nach Anspruch 1, **dadurch gekennzeichnet, dass** das Zeichen aus einer Zahl mit einer Ziffer besteht, der ein "+"-Zeichen oder ein "-"-Zeichen vorausgeht oder nachfolgt.

## Revendications

1. Plaquette orthodontique comportant une base de retenue (B), pour une dent correspondante, et des indices d'identification concernant la dent pour laquelle l'élément de correction orthodontique convient, lesdits indices consistent en un sign prévu sur la face arrière de la base de retenue (B), **caractérisés en ce que** ladite base de retenue comporte une zone de marquage d'au moins 3 millimètres carrés, **en ce que** ledit sign est produit au moyen d'un dispositif laser et qui s'étend à travers la structure en forme de réseau maillé de la base de retenue.

2. Plaquette orthodontique suivant !a revendication 1, **caractérisée en ce que** le sign consiste en un nombre comportant deux chiffres.

3. Plaquette orthodontique suivant la revendication 1, **caractérisée en ce que** le sign consiste en un nombre comportant un chiffre procédé ou suivi du signe « + », soit du sign « -«.
